(19) Europäisches Patentamt
European Patent Office
Office européen des brevets

(11) Veröffentlichungsnummer: **0 127 047**
**A2**

(12) # EUROPÄISCHE PATENTANMELDUNG

(21) Anmeldenummer: 84105436.4

(22) Anmeldetag: 14.05.84

(51) Int. Cl.³: C 07 D 493/14
C 07 D 495/14, C 07 D 493/04
A 01 N 43/90
//(C07D493/14, 311/00, 311/00, 231/00), (C07D495/14, 333/00, 311/00, 231/00), (C07D493/04, 311/00, 231/00)

(30) Priorität: 25.05.83 DE 3318876

(43) Veröffentlichungstag der Anmeldung:
05.12.84 Patentblatt 84/49

(84) Benannte Vertragsstaaten:
CH DE FR GB IT LI

(71) Anmelder: BAYER AG
Konzernverwaltung RP Patentabteilung
D-5090 Leverkusen 1 Bayerwerk(DE)

(72) Erfinder: Kühle, Engelbert, Dr.
von-Bodelschwingh-Strasse 42
D-5060 Bergisch-Gladbach 2(DE)

(72) Erfinder: Stegelmeier, Hartmut, Dr.
Meide 1 d
D-4010 Hilden(DE)

(72) Erfinder: Brandes, Wilhelm, Dr.
Eichendorffstrasse 3
D-5653 Leichlingen 1(DE)

(72) Erfinder: Hänssler, Gerd, Dr.
Heymannstrasse 40
D-5090 Leverkusen 1(DE)

(54) **N-Sulfenylierte Pyrano-pyrazol-Derivate.**

(57) N-Sulfenylierte Pyrano-pyrazol-Derivate der allgemeinen Formel (I)

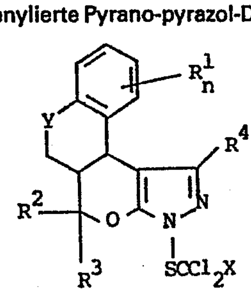

(I)

in welcher

$R^1$ für Halogen, Alkyl, Alkoxy, Alkylthio, Halogen-alkyl, Dialkylamino, Nitro oder Cyano steht,

$R^2$ und $R^3$ gleich oder verschieden sein können und für Wasserstoff, für Alkyl oder für gegebenenfalls substituiertes Phenyl stehen,

$R^4$ für Cyano, für Alkyl, für Alkoxycarbonyl oder für gegebenenfalls substituiertes Phenyl steht,

X für Halogen steht,

Y für Sauerstoff, Schwefel oder die Methylengruppe steht und

n für eine ganze Zahl von 0 bis 3 steht, sowie ihre Verwendung als Schädlingsbekämpfungsmittel.

Die neuen N-sulfenylierten Pyrano-pyrazol-Derivate können hergestellt werden, wenn man geeignete Pyrano-pyrazol-Derivate mit einem geeigneten Sulfenylchlorid umsetzt.

EP 0 127 047 A2

Croydon Printing Company Ltd.

BAYER AKTIENGESELLSCHAFT

Konzernverwaltung RP
Patentabteilung

5090 Leverkusen, Bayerwerk

BAS/ABc

Ia

## N-Sulfenylierte Pyrano-pyrazol-Derivate

Die Erfindung betrifft neue N-sulfenylierte Pyrano-pyrazol-Derivate, ein Verfahren zu ihrer Herstellung sowie ihre Verwendung als Schädlingsbekämpfungsmittel.

Es ist bereits bekannt, daß neben einer Reihe weiterer Substanzklassen auch bestimmte Heterocyclen, wie z.B. N-Trichlormethylthio-tetrahydrophthalimid, fungizide Eigenschaften besitzen (vgl. R. Wegler "Chemie der Pflanzenschutz- und Schädlingsbekämpfungsmittel" Band 2, S. 108, Springer Verlag Berlin/Heidelberg/New York 1970).

Die Wirksamkeit dieser Verbindungen ist jedoch unter bestimmten Umständen, insbesondere bei niedrigen Aufwandmengen und -konzentrationen, nicht immer völlig zufriedenstellend.

Es wurden neue N-sulfenylierte Pyrano-pyrazol-Derivate der allgemeinen Formel (I),

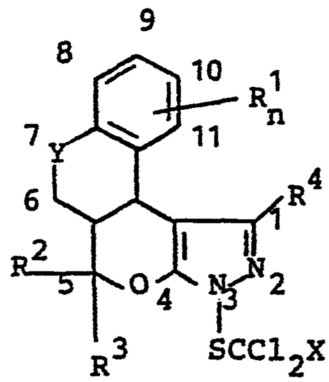 (I),

in welcher

$R^1$ für Halogen, Alkyl, Alkoxy, Alkylthio, Halogenalkyl, Dialkylamino, Nitro oder Cyano steht,

$R^2$ und $R^3$ gleich oder verschieden sein können und für Wasserstoff, für Alkyl oder für gegebenenfalls substituiertes Phenyl stehen,

$R^4$ für Cyano, für Alkyl, für Alkoxycarbonyl oder für gegebenenfalls substituiertes Phenyl steht,

X für Halogen steht,

Y für Sauerstoff, Schwefel oder die Methylengruppe steht und

n für eine ganze Zahl von 0 bis 3 steht,

gefunden.

Weiterhin wurde gefunden, daß man die neuen N-sulfenylierten Pyrano-pyrazol-Derivate der allgemeinen Formel (I)

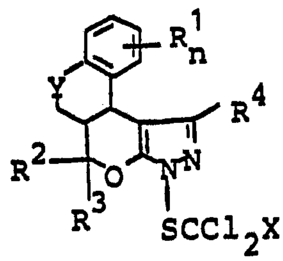

(I),

in welcher

| | |
|---|---|
| $R^1$ | für Halogen, Alkyl, Alkoxy, Alkylthio, Halogenalkyl, Dialkylamino, Nitro oder Cyano steht, |
| $R^2$ und $R^3$ | gleich oder verschieden sein können und für Wasserstoff, für Alkyl oder für gegebenenfalls substituiertes Phenyl stehen, |
| $R^4$ | für Cyano, für Alkyl, für Alkoxycarbonyl oder für gegebenenfalls substituiertes Phenyl steht, |
| X | für Halogen steht, |
| Y | für Sauerstoff, Schwefel oder die Methylengruppe steht und |
| n | für eine ganze Zahl von 0 bis 3 steht, |

erhält, wenn man Pyrano-pyrazole der allgemeinen Formel (II)

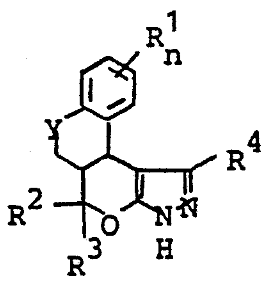

(II),

in welcher

$R^1$, $R^2$, $R^3$, $R^4$, Y und n die weiter oben angegebene Bedeutung haben,

mit einem Sulfenylchlorid der allgemeinen Formel (III)

$$XCl_2CSCl \qquad (III),$$

in welcher

X die oben angegebene Bedeutung hat,

in Gegenwart eines säurebindenden Mittels und gegebenenfalls in Gegenwart eines Verdünnungsmittels umsetzt.

Die neuen N-sulfenylierten Pyrano-pyrazol-Derivate der allgemeinen Formel (I) zeichnen sich durch hohe Wirksamkeit als Schädlingsbekämpfungsmittel, insbesondere durch ihre hervorragende fungizide Wirkung, aus.

Die erfindungsgemäßen Stoffe stellen somit eine Bereicherung der Technik dar.

Die erfindungsgemäßen N-sulfenylierten Pyrano-pyrazol-Derivate sind durch die Formel (I) allgemein definiert.

Die Alkylreste in $R^1$, $R^2$, $R^3$ und $R^4$ sowie der Alkoxyteil des Alkoxycarbonyl-Restes in $R^4$ können geradkettig oder verzweigt sein und enthalten vorzugsweise jeweils 1 bis 6, insbesondere 1 bis 4 und besonders bevorzugt 1 oder 2 Kohlenstoffatome. Die Alkylteile der Alkoxy,

Alkylthio, Halogenalkyl und Dialkylamino-Reste in $R^1$ können geradkettig oder verzweigt sein und enthalten vorzugsweise jeweils 1 bis 4, insbesondere 1 bis 3 und besonders bevorzugt 1 oder 2 Kohlenstoffatome. Der Halogenalkylteil des genannten Restes enthält vorzugsweise 1 bis 7, insbesondere 1 bis 5 gleiche oder verschiedene Halogenatome, wie Fluor, Chlor, Brom oder Jod, vorzugsweise Fluor und/oder Chlor.

Beispielhaft seien für die genannten Gruppen die folgenden Reste aufgeführt: Methyl, Ethyl, n-Propyl, i-Propyl, n-Butyl, i-Butyl, sec-Butyl, tert.-Butyl, Methoxy, Ethoxy, n-Propoxy, i-Propoxy, n-Butoxy, i-Butoxy, sec-Butoxy, tert.-Butoxy, Methylthio, Ethylthio, n-Propylthio, i-Propylthio, n-Butylthio, i-Butylthio, sec-Butylthio, tert.-Butylthio, Trifluormethyl, Trichlormethyl, Dichlorfluormethyl, Chlordifluormethyl, Difluormethyl, Trifluorethyl, Trichlorethyl, Dichlorfluorethyl, Chlordifluorethyl, Difluorethyl, Dimethylamino, Diethylamino, Di-n-propylamino, Methoxycarbonyl, Ethoxycarbonyl, n-Propoxycarbonyl und iso-Propoxycarbonyl. Halogen bedeutet, wo nicht anders erläutert, Fluor, Chlor, Brom und Jod, vorzugsweise Chlor, Brom und Fluor.

Als Phenylsubstituenten in $R^2$, $R^3$ und $R^4$ seien Halogen und/oder Alkyl genannt mit der oben gegebenen Bedeutung dieser Reste.

Bevorzugt sind diejenigen Verbindungen der Formel (I), bei denen

$R^1$ für Halogen, Cyano, Nitro, für geradkettiges oder verzweigtes Alkyl mit bis zu 4 Kohlenstoffatomen, für Alkoxy, Alkylthio oder Dialkylamino mit 1 oder 2 Kohlenstoffatomen in den jeweiligen Alkylteilen oder für Halogenalkyl mit 1 oder 2 Kohlenstoffatomen und bis zu 5 gleichen oder verschiedenen Halogenatomen, wie Monohalogenalkyl, Dihalogenalkyl, Trihalogenalkyl, Tetrahalogenalkyl oder Pentahalogenalkyl, steht und

$R^2$ und $R^3$, welche gleich oder verschieden sein können, für Wasserstoff, für geradkettiges oder verzweigtes Alkyl mit bis zu 4 Kohlenstoffatomen oder für gegebenenfalls einfach bis fünffach, gleich oder verschieden durch Halogen oder Alkyl mit bis zu 4 Kohlenstoffatomen substituiertes Phenyl stehen,

$R^4$ für Cyano, für geradkettiges oder verzweigtes Alkyl oder Alkoxycarbonyl mit jeweils bis zu 4 Kohlenstoffatomen im Alkylteil oder für gegebenenfalls einfach bis fünffach, gleich oder verschieden durch Halogen oder Alkyl mit bis zu 4 Kohlenstoffatomen substituiertes Phenyl steht,

X für Chlor oder Fluor steht,

Y für Sauerstoff, Schwefel oder die Methylengruppierung steht und

n für eine ganze Zahl von 0 bis 3 steht.

Ganz besonders bevorzugt sind diejenigen Verbindungen der Formel (I), bei denen

$R^1$ für Fluor, Chlor, Brom, Cyano, Nitro, Methyl, Methoxy, Methylthio, Dimethylamino sowie Trifluormethyl steht und

$R^2$ und $R^3$, welche gleich oder verschieden sein können, für Wasserstoff, für Methyl oder für gegebenenfalls ein bis dreifach, gleich oder verschieden durch Chlor oder Methyl substituiertes Phenyl stehen,

$R^4$ für Methyl, Ethyl, n- und i-Propyl, t-Butyl, Cyano, Methoxycarbonyl, Ethoxycarbonyl sowie gegebenenfalls ein- bis dreifach, gleich oder verschieden durch Chlor oder Methyl substituiertes Phenyl steht,

X für Chlor oder Fluor steht,

Y für Sauerstoff, Schwefel oder die Methylengruppierung steht und

n für eine ganze Zahl von 0 bis 2 steht.

Im einzelnen seien außer den bei den Herstellungsbeispielen genannten Verbindungen die folgenden Verbindungen der allgemeinen Formel (I) genannt:

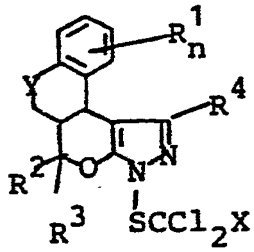

(I)

| $R^2$ | $R^3$ | $R^4$ | $R^1$ | Y | n | X |
|---|---|---|---|---|---|---|
| $CH_3$ | $CH_3$ | $CH_3$ | - | $-CH_2-$ | 0 | F |
| $CH_3$ | $CH_3$ | $CH_3$ | 8,11-$Cl_2$ | S | 2 | F |
| $CH_3$ | $CH_3$ | CN | - | O | 0 | Cl |
| $CH_3$ | $CH_3$ | CN | - | O | 0 | F |
| $CH_3$ | $CH_3$ | $CH_3$ | - | S | 0 | F |
| $CH_3$ | $CH_3$ | $CH_3$ | 10-$NO_2$ | $-CH_2-$ | 1 | F |
| $CH_3$ | $CH_3$ | $CH_3$ | 8,11-$Cl_2$ | $-CH_2-$ | 2 | F |

Verwendet man beispielsweise 1,5,5-Trimethyl-5a,11b-dihydro-3H, 5H, 6H, 4,7-dioxa-2,3- diazacyclopenta-(c)-phenanthren und Dichlorfluormethansulfenylchlorid als Ausgangsstoffe, so kann der Reaktionsverlauf des erfindungsgemäßen Verfahrens durch das folgende Formelschema wiedergegeben werden:

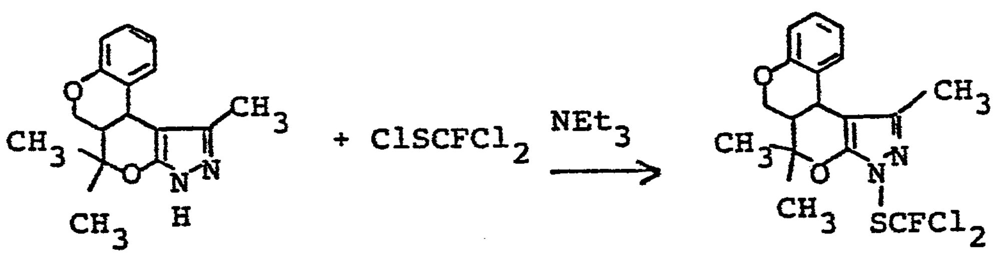


Die zur Durchführung des erfindungsgemäßen Verfahrens benötigten Pyranopyrazole sind durch die Formel (II) allgemein definiert.

Ihre Herstellung erfolgt gemäß einem nicht zum Stand der Technik gehörenden, d.h. noch nicht vorveröffent-

0127047

lichten Verfahren aus substituierten aromatischen Aldehyden der allgemeinen Formel (IV),

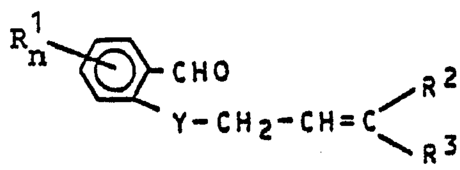

(IV)

in welcher

$R^1$, $R^2$, $R^3$, Y und n die oben angegebene Bedeutung haben,

mit Pyrazolinonen der allgemeinen Formel (V),

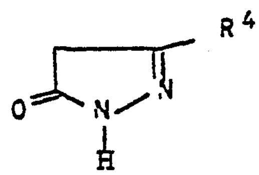

(V),

in welcher

$R^4$ die oben angegebene Bedeutung hat,

in Gegenwart eines Verdünnungsmittels und gegebenenfalls in Gegenwart eines Katalysators.

Die substituierten aromatischen Aldehyde der Formel (IV) sind bekannt (vgl. z.B. "Chem. Pharm. Bull." 27, 2943 (1979) oder BE-PS 816 463 oder "Liebigs Ann. Chem." 401, 21 (1913)).

Ebenso sind die Pyrazolinone der Formel (V) bekannt

(vgl. z.B. "The Chemistry of Heterocyclic Compounds" Vol. 20, Wiley, New York 1964).

Die weiterhin zur Durchführung des erfindungsgemäßen Verfahrens benötigten, bekannten Sulfenylchloride sind durch die Formel (III) allgemein definiert.

Im einzelnen können Dichlorfluormethan- und Trichlormethansulfenylchlorid eingesetzt werden.

Bei der Durchführung des Verfahrens kommen als Verdünnungsmittel alle inerten organischen Lösungsmittel in Frage. Hierzu gehören vorzugsweise Kohlenwasserstoffe, wie Toluol, Chlorkohlenwasserstoffe, wie Methylenchlorid und Chlorbenzol, oder Ether wie Dioxan. Man kann die Umsetzung aber auch im wäßrigen Medium durchführen.

Als säurebindende Mittel können anorganische Basen, wie Natriumhydroxid und Natriumcarbonat oder tert. Amine, wie Pyridin und Triethylamin verwendet werden.

Die Reaktionstemperaturen können in einem größeren Bereich variiert werden. Im allgemeinen arbeitet man von 0°C bis 100°C, vorzugsweise von 20 bis 50°C.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

So werden z.B. fungizide Mittel im Pflanzenschutz eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Als Pflanzenschutzmittel können die erfindungsgemäßen Wirkstoffe mit besonders gutem Erfolg zur Bekämpfung von Botrytis-Arten, wie z.B. gegen den Erreger des Grauschimmels (Botrytis cinerea), eingesetzt werden. Daneben können sie auch zur Bekämpfung von Reiskrankheiten, wie z.B. gegen Pyricularia und Pellicularia sasakii, eingesetzt werden.

Die Wirkstoffe können in die üblichen Formulierungen übergeführt werden, wie Lösungen, Emulsionen, Spritzpulver, Suspensionen, Pulver, Stäubemittel, Schäume, Pasten, lösliche Pulver, Granulate, Aerosole, Suspensions-Emulsionskonzentrate, Saatgutpuder, Wirkstoffimprägnierte Natur- und synthetische Stoffe, Feinstverkapselungen in polymeren Stoffe und in Hüllmassen für Saatgut, ferner in Formulierungen mit Brennsätzen, wie Räucherpatronen, -dosen, -spiralen u.ä. sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage:

z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen oder in den verschiedenen Anwendungsformen in Mischung mit anderen bekannten Wirkstoffen vorliegen, wie Fungiziden, Bakteriziden, Insektiziden, Akariziden, Nematiziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder der daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,02 Gew.-%, am Wirkungsort erforderlich.

Das erfindungsgemäße Verfahren soll durch die folgenden Herstellungsbeispiele erläutert werden:

## Herstellungsbeispiele

### Beispiel 1

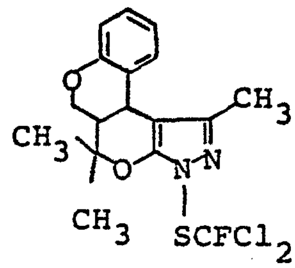

13,6 g (0,05 Mol) 1,5,5-Trimethyl-5a,11b-dihydro-3H, 5H, 6H, 4,7-dioxa-2,3-diazacyclopenta-(c)-phenanthren werden unter Zusatz von 6 g (0,06 Mol) Triethylamin in 100 ml Dioxan verrührt. Bei ca.20°C tropft man hierzu 8,5 g (0,05 Mol) Dichlorfluormethansulfenylchlorid; hierbei steigt die Temperatur bis etwa 50°C an. Man rührt den Kristallbrei etwa 1 h, setzt Wasser zu und saugt das Reaktionsprodukt ab. Man wäscht mit Methanol nach und trocknet bei 50°C. Man erhält 15 g an 1,5,5-Trimethyl-3-dichlorfluormethylthio-5a,11b-dihydro-3H, 5H, 6H, 4,7-dioxa-2,3-diazacyclopenta(c)phenanthren vom Schmelzpunkt 178-180°C.

In analoger Weise können die folgenden Verbindungen der Formel I hergestellt werden:

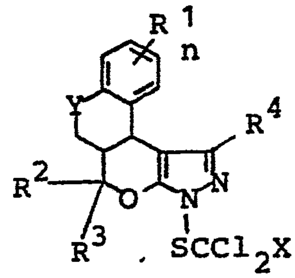

(I)

| Bsp. Nr. | X | Y | $R^1$ | n | $R^2$ | $R^3$ | $R^4$ | Schmelzpunkt [°C] |
|---|---|---|---|---|---|---|---|---|
| 2 | Cl | 0 | - | 0 | $CH_3$ | $CH_3$ | $CH_3$ | 158 |
| 3 | F | 0 | 10-Br | 1 | $CH_3$ | $CH_3$ | $CH_3$ | 100 |
| 4 | F | 0 | 8,10-$Cl_2$ | 2 | $CH_3$ | $CH_3$ | $CH_3$ | 112 |
| 5 | F | 0 | 9-$CH_3O$ | 1 | $CH_3$ | $CH_3$ | $CH_3$ | 95 |
| 6 | F | 0 | 8-$CH_3O$ | 1 | $CH_3$ | $CH_3$ | $CH_3$ | 137 |
| 7 | F | S | - | 0 | $CH_3$ | $CH_3$ | $CH_3$ | 95-100 |

Anwendungsbeispiele

In den folgenden Anwendungsbeispielen werden die nachstehend aufgeführten Verbindungen als Vergleichssubstanzen eingesetzt:

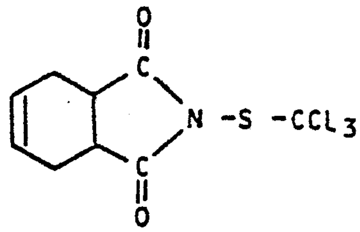

(A)

(N-Trichlormethylthio-tetrahydrophthalimid)

und

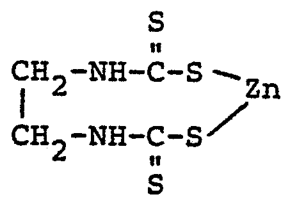

(B)

(Zinkethylenbisdithiocarbamat)

Beispiel A

Botrytis-Test (Bohne)/protektiv

Lösungsmittel: 4,7 Gewichtsteile Aceton
Emulgator: 0,3 Gewichtsteile Alkyl-aryl-polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten, feuchten Kammer bei 20°C aufgestellt. 3 Tage nach der Inokulation wird die Größe der Befallsflecken auf den Blättern ausgewertet.

Eine deutliche Überlegenheit in Wirksamkeit gegenüber dem Stand der Technik zeigen in diesem Test z.B. die Verbindungen gemäß folgender Herstellungsbeispiele: 1,3,4 und 5.

Beispiel B

Pyricularia-Test (Reis) /protektiv

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25°C aufgestellt.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäß folgender Herstellungsbeispiele: 4,6 und 7.

0127047

## Beispiel C

Pyricularia-Test (Reis) /systemisch

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und verdünnt das Konzentrat mit Wasser und der angegebenen Menge Emulgator auf die gewünschte Konzentration.

Zur Prüfung auf systemische Eigenschaften werden 40 ml der Wirkstoffzubereitung auf Einheitserde gegossen, in der junge Reispflanzen angezogen wurden. 7 Tage nach der Behandlung werden die Pflanzen mit einer wäßrigen Sporensuspension von Pyricularia oryzae inokuliert. Danach verbleiben die Pflanzen in einem Gewächshaus bei einer Temperatur von 25°C und einer rel. Luftfeuchtigkeit von 100 % bis zur Auswertung.

4 Tage nach der Inokulation erfolgt die Auswertung des Krankheitsbefalls.

Eine deutliche Überlegenheit in der Wirksamkeit gegenüber dem Stand der Technik zeigen in diesem Test z.B. die Verbindungen gemäß folgender Herstellungsbeispiele: 5,6 und 7.

## Patentansprüche

1. N-Sulfenylierte Pyrano-pyrazol-Derivate der allgemeinen Formel (I)

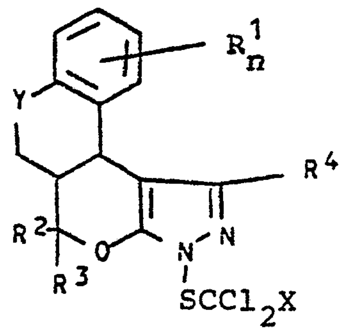

(I)

in welcher

$R^1$ für Halogen, für Alkyl, für Alkoxy, Halogenalkyl, Alkylthio, Dialkylamino, Nitro oder Cyano steht,

$R^2$ und $R^3$ gleich oder verschieden sein können und für Wasserstoff, für Alkyl oder für gegebenenfalls substituiertes Phenyl stehen,

$R^4$ für Cyano, für Alkyl, für Alkoxycarbonyl oder für gegebenenfalls substituiertes Phenyl steht,

X für Halogen steht,

Y für Sauerstoff, Schwefel oder die Methylengruppe steht, und

n für eine ganze Zahl von 0 bis 3 steht.

0127047

2. N-Sulfenylierte Pyrano-pyrazol-Derivate gemäß Anspruch 1, wobei in der Formel (I)

$R^1$ für Halogen, Cyano, Nitro, für geradkettiges oder verzweigtes Alkyl mit bis zu 4 Kohlenstoffatomen, für Alkoxy, Alkylthio oder Dialkylamino mit 1 oder 2 Kohlenstoffatomen in den jeweiligen Alkylteilen oder für Monohalogenalkyl, Dihalogenalkyl, Trihalogenalkyl, Tetrahalogenalkyl oder Pentahalogenalkyl, steht und

$R^2$ und $R^3$, welche gleich oder verschieden sein können, für Wasserstoff, für geradkettiges oder verzweigtes Alkyl mit bis zu 4 Kohlenstoffatomen oder für gegebenenfalls einfach bis fünffach, gleich oder verschieden durch Halogen oder Alkyl mit bis zu 4 Kohlenstoffatomen substituiertes Phenyl stehen,

$R^4$ für geradkettiges oder verzweigtes Alkyl oder Alkoxycarbonyl mit jeweils bis zu 4 Kohlenstoffatomen im Alkylteil, für Cyano oder für gegebenenfalls einfach bis fünffach, gleich oder verschieden durch Halogen oder Alkyl mit bis zu 4 Kohlenstoffatomen substituiertes Phenyl steht,

X für Chlor oder Fluor steht,

Y für Sauerstoff, Schwefel oder die Methylengruppierung steht und

n für eine ganze Zahl von 0 bis 3 steht.

3. N-Sulfenylierte Pyrano-pyrazol-Derivate der allgemeinen Formel (I) gemäß Anspruch 1, wobei

$R^1$ für Fluor, Chlor, Brom, Cyano, Nitro, Methyl, Methoxy, Methylthio, Dimethylamino sowie Trifluormethyl steht,

$R^2$ und $R^3$, welche gleich oder verschieden sein können, für Wasserstoff, für Methyl oder für gegebenenfalls ein- bis dreifach, gleich oder verschieden durch Chlor oder Methyl substituiertes Phenyl stehen,

$R^4$ für Methyl, Ethyl, n- und i-Propyl, t-Butyl, Cyano, Methoxycarbonyl, Ethoxycarbonyl sowie gegebenenfalls ein- bis dreifach, gleich oder verschieden durch Chlor oder Methyl substituiertes Phenyl steht,

X für Chlor oder Fluor steht,

Y für Sauerstoff, Schwefel oder die Methylengruppierung steht und

n für eine ganze Zahl von 0 bis 2 steht.

4. Verfahren zur Herstellung von N-sulfenylierten Pyrano-pyrazol-Derivaten der allgemeinen Formel (I)

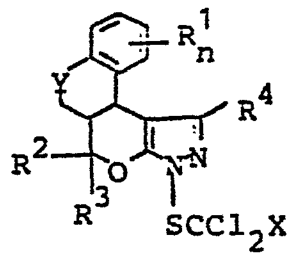

(I)

in welcher

$R^1$ für Halogen, Alkyl, Alkoxy, Alkylthio, Halogenalkyl, Dialkylamino, Nitro oder Cyano steht und

$R^2$ und $R^3$ gleich oder verschieden sein können und für Wasserstoff, für Alkyl oder für gegebenenfalls substituiertes Phenyl stehen,

$R^4$ für Cyano, für Alkyl, für Alkoxycarbonyl oder für gegebenenfalls substituiertes Phenyl steht,

X für Halogen steht,

Y für Sauerstoff, Schwefel oder die Methylengruppe steht und

n für eine ganze Zahl von 0 bis 3 steht,

dadurch gekennzeichnet, daß man Pyrano-pyrazole der allgemeinen Formel (II)

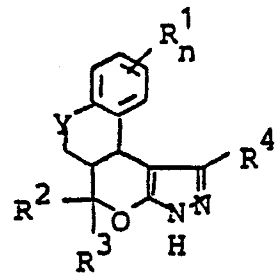

(II)

in welcher

$R^1$, $R^2$, $R^3$, $R^4$, Y und n die weiter oben angegebene Bedeutung haben,

mit einem Sulfenylchlorid der allgemeinen Formel (III)

$$XCl_2CSCl \quad (III)$$

in welcher

X die oben angegebene Bedeutung hat,

in Gegenwart eines säurebindenden Mittels und gegebenenfalls in Gegenwart eines Verdünnungsmittels, umsetzt.

5. Schädlingsbekämpfungsmittel, gekennzeichnet durch einen Gehalt an mindestens einem N-sulfenylierten Pyranopyrazol-Derivat der Formel (I) in Ansprüchen 1 und 4.

6. Verfahren zur Bekämpfung von Schädlingen, dadurch gekennzeichnet, daß man N-sulfenylierte Pyrano-pyrazol-Derivate der Formel (I) in Ansprüchen 1 und 4 auf Schädlinge oder ihren Lebensraum einwirken läßt.

7. Verwendung von N-sulfenylierten Pyrano-pyrazol-Derivaten der Formel (I) in Ansprüchen 1 und 4 als Schädlingsbekämpfungsmittel.

8. Verwendung von N-sulfenylierten Pyrano-pyrazol-Derivaten der Formel (I) in Ansprüchen 1 und 4 zur Bekämpfung von Schädlingen.

9. Verfahren zur Herstellung von Schädlingsbekämpfungsmitteln, dadurch gekennzeichnet, daß man N-sulfenylierte Pyrano-pyrazol-Derivate der Formel (I) in Ansprüchen 1 und 4 mit Streckmitteln und/oder oberflächenaktiven Mitteln vermischt.